# EUROPÄISCHE PATENTSCHRIFT

(11) **EP 1 641 407 B1**
(45) Veröffentlichungstag und Bekanntmachung des Hinweises auf die Patenterteilung: **24.01.2007**
(21) Anmeldenummer: 04738762.6
(22) Anmeldetag: 23.06.2004
(51) Int. Cl.: A61B 19/00, A61B 19/08, A61B 19/10

(54) **CHIRURGISCHE ABDECKTÜCHER**
SURGICAL COVERS
CHAMPS OPERATOIRES

(30) Priorität: 26.06.2003 DE 10328907
(43) Veröffentlichungstag der Anmeldung: 05.04.2006
(73) Patentinhaber: Philipps-Universität Marburg, 35032 Marburg (DE)
(72) Erfinder: JUNK, Klaus, 35041 Marburg (DE)
(74) Vertreter: Buchhold, Jürgen
(86) Internationale Anmeldenummer: PCT/DE2004/001313
(87) Internationale Veröffentlichungsnummer: WO 2005/002456

(56) Entgegenhaltungen:
- WO-A-01/30258
- US-A- 4 027 665
- US-A- 5 490 524
- US-A- 5 503 163
- US-A- 5 778 889

## Beschreibung

Die Erfindung betrifft ein chirurgisches Abdecktuch, insbesondere für Kopfoperationen.

Chirurgische Abdecktücher dienen dazu den Bereich des chirurgischen Eingriffs so sauber und steril wie möglich zu halten. Die Abdecktücher weisen dazu meist dem Eingriffsbereich entsprechend geformte Öffnungen in der Hauptbahn der Abdecktücher, sogenannte "Fenestrationen" auf, oder werden entsprechend zugeschnitten. Innerhalb der Fenestration werden dann sogenannte "Einschneidefolien" (Inzissionsfolien oder incise drapes/foils) eingeklebt. Von einigen Herstellern können solche Abdecktücher mit bereits eingefügter Einschneidefolie in standardisierten Massen auch bereits konfektioniert bezogen werden.

Nachdem die Haut des Patienten im Eingriffsbereich gesäubert und desinfiziert wurde, wird die Einschneidefolie gewöhnlich auf die Haut im Bereich des chirurgischen Eingriffs aufgeklebt und gestatten -da die Folien meist durchsichtig ausgeführt sind- einen direkten Sichtkontakt auch mit dem Randbereich des chirurgischen Eingriffsbereiches. Zur Fixierung verfügen die Einschneidefolien über besondere Klebebereiche, welche gewöhnlich durch abziehbare Schutzfolien bedeckt sind.

Im Stand der Technik sind einige solcher Abdecktücher, umfassend "Einschneide-Tücher oder -folien) bekannt.

So zeigt z.B. die DE 26 02 562 A1 (basierend auf US 545 288) und die DE 26 02 563 A1 (basierend auf US 545 289) (mit Anmelder Johnson & Johnson und Prioritätstag 29.1.1975) jeweils ein chirurgisches Abdecktuch bestehend aus einer Hauptbahn, bestehend aus einem biegsamen anpassungsfähigen Material, einer im oberen Bereich der Hauptbahn darauf angebrachte Manschette mit Taschen zur Aufnahme von Händen und einem Klebstoffstreifen, um das Abdecktuch entfernbar am Köper (insbesondere Kopf) des Patienten zu befestigen. Beide vorgenannten Tücher weisen mehrere komplexe Faltlinien auf. Beide dienen dem Zweck die Vorbereitung einer Operation zu verkürzen und eine sterile Abdeckung zu gewährleisten.

Die US PS 37 91 382 weist, zitiert in der US PS 29 15 627 (Johnson & Johnson, Anmeldetag 18.4.79), weist an der Oberfläche eines solchen Abdecktuches, in dessen Bereich der vom Operationstisch herunter hängt, Beutel zur Aufnahme von Flüssigkeiten auf, welche typischerweise während einer Operation am Kopf auftreten. Dazu schlägt die US PS 29 15 627 eine Verbesserung in Form von besonders geformten Lappen vor, die dafür sorgen sollen, eventuell auftretende Flüssigkeiten tatsächlich sicher in einen solchen Beutel zu führen.

Eine jüngere Schrift (US 6 129 085, als divisional application von Ser. No. 08/705,689 angemeldet am 30.8.1996, nun US Pat. No. 5 778 889, Kimberly-Clark) schlägt ebenfalls ein chirurgisches Abdecktuch (Craniotomy drape entspr. Schädeleröffnungs-Tuch) insbesondere für Operationen am Schädel vor. Das dort vorgestellte Abdecktuch soll dem Narkose-Arzt jederzeit während der Operation einen direkten Blickkontakt zum Gesicht des Patienten ermöglichen, ferner soll das Tuch nicht auf dem Boden des Operationssaals schleifen und schließlich soll das Tuch einfach einrichtbare und tragkräftige Klemmen zur Halterung von Werkzeugen, Schläuchen etc. aufweisen. Bei allen Aufgaben soll die Sterilität des Raumes im Bereich des Patienten gewahrt bleiben. Zur Lösung dieser Aufgabe schlägt die obige US 5 778 889, ein Abdecktuch ("craniotomy drape") mit einem Auffangteil für vor, dass an den Seiten einer Hauptbahn einen "Zwickel" oder "Ecktuch" ("gusset") sowie in einem Ausführungsbeispiel ebenfalls seitlich durchsichtige, ebene Teilstücke anzubringen. Beide Teile (Zwickel und durchsichtige Teilstücke) sind so bemessen, dass der Boden des Operationssaales (in Abhängigkeit von der Höhe des OP-Tisches) nicht berührt wird. Ferner weist das Abdecktuch in einem anderen Ausführungsbeispiel Halterungen zur Aufnahme von Werkzeugen bzw. Kabeln und Schläuchen auf.

Die DE 195 10 020 A1 (General Electric, Anmeldedatum 29.3.94 von US 2 195 79) schlägt ein Abdecktuch vor, dass zwischen zwei Schichten eine Spule umfasst, so dass während Operationen ein MRI-Aufnahme gemacht werden kann.

Schriften, die sich direkt und nahezu ausschliesslich mit "Einschneide-Tüchern" oder -Folien (Incise Sheets, drapes etc.) sind z.B.:
CA 2 314 962 (Anmeldedatum 16.10.1998) schlägt besondere, punkt- und ringförmige Anordnung der Klebeflächen vor.
US 5 979 450 (Anmelder 3M, Anmeldedatum 9.4.1998) stellt eine Inzissions- (oder Einschneide-)folie oder -tuch zur
Verfügung, das durch den Einsatz von Abdeckbahnen und ggf. zusätlichen Versteifungsbahnen ("liners") versteift wird, um die Einschneidefolie faltenfrei am Körper des Patienten anbringen zu können.
EP 0 902 660 B1 (Anmelder 3M, Anmeldedatum aus den prioritätsbegründenden Anmeldungen US 6 489 03 und 7 2 47 44, früheste 16.5.1996) schlägt mit dem gleichen Ziel eine abrollbare Einschneidefolie ohne Abdeckbahnen (liners) vor.
EP 0 568 401 B1 (Anmelder Laboratoire Hydrex (SA), FR, Anmeldedatum mir Priorität der FR 92 052 50, 29.4.92) schlägt mit dem gleichen Ziel der "faltenfreien Anbringbarkeit" eine ebenfalls (wie EP 0 902 660 B1) dreischichtige Einschneidefolie vor, die aber eine Abdeckbahn zur Versteifung auf der anderen Seite der durchsichtigen Folie aufweist und zu dem eine Perforation durch alle drei Schichten aufweist.

Ein chirurgisches Abdecktuch gemäß dem Oberbegriff des Anspruchs 1 ist aus der WO-A-01/302 58 bekannt.

Aufgrund der weiteren Entwicklung innerhalb der Chirurgie wird die genaue Lage des chirurgischen Eingriffsbereiches mittlerweile vor dem Eingriff 2- oder 3-dimensional (z.B. durch MRI, MRT, Röntgen oder Ultraschall) vermessen, um den Bereich so genau wie möglich zu lokalisieren und damit z.B. gesundes Gewebe möglichst zu schonen.

Nach dieser "Vermessung" erfolgt dann der chirurgische Eingriff im OP (Operationssaal), wobei zur Beibehaltung der Kenntnis der genauen Lage des chirurgischen Eingriffsbereiches weitere oder andere dreidimensionale Vermessungssysteme, z.B. sogenannte Navigationssysteme, die z.B. mit zwei Infrarot-Sendern und Empfängern arbeiten, eingesetzt werden. Insgesamt wird damit ein Wechsel des Koordinaten-Bezugspunktes für den chirurgischen Eingriffsbereich notwendig. Zur Durchführung dieses Wechsels wird daher meist im Bereich des chirurgischen Eingriffs am Operationstisch oder einer anderen Stelle im OP mit festem Bezug zum chirurgischen Eingriffsbereich ein irgendwie geometrisch geeignet geformtes und vom zweiten Vermessungssystem (im Folgenden Navigations-system genannt) identifizierbares Bezugsmittel angebracht. Das zweite Vermessungssystem (Navigations-System) muss dabei nicht notwendigerweise anders als das erste Vermessungssystem sein.

So werden z.B. mittlerweile häufig 2 oder 3-dimensionale Navigationssysteme mit der entsprechenden Anzahl von z.B. Infrarot-Sendern und -Empfängern eingesetzt, welche ein Bezugsmittel, z.B. in Form eines dreiarmigen Sterns (genannt Navigationsbasiseinheit) umfassen.

Durch "Übereinanderschieben" des Bildes des Eingriffsbereiches hergestellt durch das erste und durch das zweite Vermessungssystem, z.B. an einem geeigneten Monitor, wird dann der Wechsel des Koordinaten-Bezugspunktes vollzogen. Um den Bezug der Lage des Eingriffsbereiches während der Operation zur Lage des Eingriffsbereiches während der ersten, meist genaueren Vermessung, nicht zu verlieren ist es daher notwendig, dass das Navigationssystem auch während der Operation mehr oder weniger permanent in Wechselwirkung zum Bezugsmittel steht, so dass das Bezugsmittel möglichst ausserhalb des Bereiches der Bewegung der Operateure angebracht werden sollte. Andererseits sind die Bezugsmittel vorteilhafterweise in der Nähe des Eingriffsbereiches anzubringen.

Als Kompromiss ergibt sich daher die Notwendigkeit neben dem Eingriffsbereich auch die Bezugsmittel möglichst steril, aber für das Navigationssystem identifizierbar abzudecken. Diese Aufgabe wird in den Fällen erschwert, in denen das Bezugsmittel -aus Gründen der Arbeitsergonomie für das OP-Team- in einer Ebene angebracht wird, der ausserhalb des Arbeitsbereiches des OP-Teams und damit meist oberhalb des Eingriffsbereiches liegt.

Im Stand der Technik, insbesondere aus den vorgenannten Schriften, ist dazu keine praktikable Lösung bekannt. Im Stand der Technik sind lediglich solche chirurgischen Abdecktücher bekannt die neben einer Fenestration für eine Einschneidefolie noch einen -einseitig am Abdecktuch befestigten- flüssigkeitsundurchlässigen Beutel im Bereich zwischen oberem Rand der Einschneidefolie und oberem Ende des Abdecktuches aufweisen. Darüber hinaus weisen die bekannten Abdecktücher meist noch Versteifungsmittel an den seitlichen Rändern der Einschneidefolie und im Einlassbereich des flüssigkeitsundurchlässigen Beutels auf. Meist dienen diese Versteifungsmittel, z.B. in Form von Blechstreifen, dazu eine Führung für während der Operation auftretende Flüssigkeiten oder Operationsrückstände auszubilden, so diese insgesamt in dem Beutel zu liegen kommen.

### [Aufgabe der Erfindung]

Aufgabe der Erfindung ist es daher ein neues chirurgisches Abdecktuch vorzusehen, welches neben der sterilen Bedeckung des chirurgischen Eingriffsbereiches auch die sterile Bedeckung irgendwie geformter Bezugsmittel für bekannte Vermessungssysteme, insbesondere die Infrarotstrahlung nutzende Vermessungssysteme, ermöglicht.

### [Beispiele]

Diese Aufgabe wird gelöst durch den Gegenstand des Patentanspruchs 1.

Zur Lösung wird ein chirurgisches Abdecktuch mit einer ersten Fenestration für eine Einschneidefolie mit oder ohne einem dieser Folie zugeordneten Auffangbeutel für Operationsrückstände nach dem Stand der Technik, dadurch gekennzeichnet, dass das Abdecktuch mindestens eine weitere Fenestration aufweist, zur Aufnahme eines Abdeckmittels für ein aus der Ebene der Oberfläche des Abdecktuches herausragendes Bezugsmittel, welches für ein 2 oder 3-dimensionales Vermessungssystem identifizierbar ist.

Damit erreicht man den Vorteil, neben dem Patienten auch ein Bezugsmittel, wie es für die modernen -mit 2- oder 3-dim. Vermessungsgeräten ausgestatteten- Operationssäle üblich ist, steril abzudecken, ohne die Beweglichkeit des Bezugsmittels einzuschränken oder während der Operation die Grenze zwischen sterilem zu unsterilem Operationsbereich durchbrechen zu müssen.

Das gesamte neuartige Abdecktuch kann somit in einem Gang sterilisiert werden. Damit ergibt sich auch bei der Operationsvorbereitung ein deutlicher Zeitgewinn, welcher beispielsweise der Betreuung des Patienten gewidmet werden kann.

Weitere Vorteile ergeben sich durch den Gegenstand der jeweiligen Unteransprüche bzw. das beanspruchte Herstellungsverfahren.

Weitere Einzelheiten und vorteilhafte Ausbildungen der Erfindung ergeben sich aus den im folgenden beschriebenen und in den Zeichnungen dargestellten Ausführungsbeispiel, sowie aus den Unteransprüchen. Es zeigt:
**Fig.** 1 einen Schnitt durch das erfingunsgemäße Abdecktuch entlang der Linie I-II aus Fig. 2
**Fig.** 2 eine Aufsicht auf das erfindungsgemäße Abdecktuch mit zwei Abdeckmitteln
**Fig.** 3 einen Schnitt durch ein erfindungsgemäßes Abdeckmittel
**Fig.** 4a einen Schnitt durch ein Ausführungsbeispiel des erfindungsgemäßen Abdeckmittels
**Fig.** 4b einen Schnitt durch ein weiteres Ausführungsbeispiel des erfindungsgemäßen Abdeckmittels

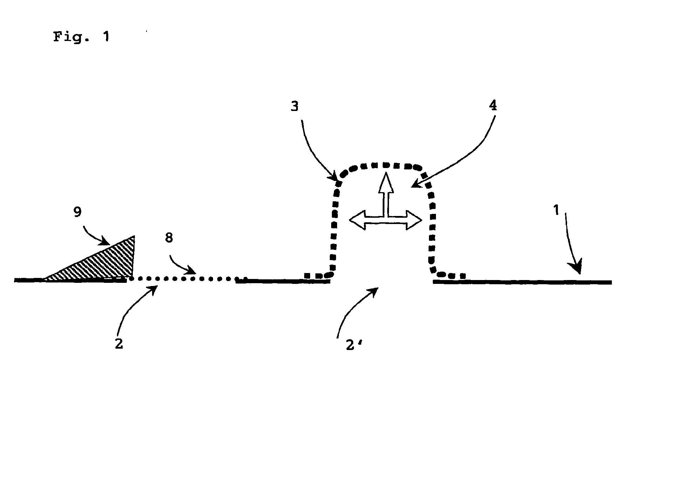

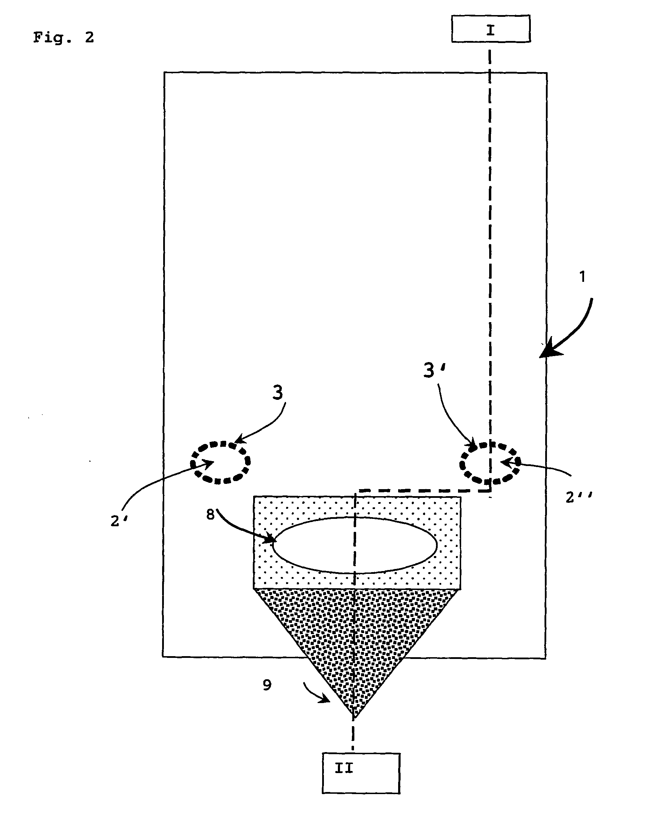

**Fig. 1** zeigt ein Ausführungsbeispiel des erfindungsmgemäßen Abdeckmittels im Schnitt durch die Linie I-II aus Fig. 2. Dabei wird das Bezugsmittel 4 vom Abdeckmitte 3, welches mit dem Abdecktuch 1 am Umfang einer zweiten Fenestration (2') verbunden ist dargestellt. Im linken Teil der Figur ist die erste Fenestration 2 für die Einschneidefolie 8 dargestellt. Darüber in Richtung Rand des Abdecktuches 1 ist der der Einschneidefolie zugeordnete Flüssigkeitsauffangbeutel 9 dargestellt.

**Fig. 2** zeigt eine Aufsicht auf das erfindungsgemäße Abdecktuch, in diesem Ausführungsbeispiel mit zwei Adeckmitteln, welche jeweils ca. 25 cm im Durchmesser messen und ca. 50 cm vom Zentrum der Einschneidefolie entfernt angebracht angeordnet sind. Dadurch ist ein Umschwenken des Bezugsmittels auf die andere Seite des OP-Tisches auch während einer Operation leicht durchführbar.

Ebenfalls dargestellt ist die Einschneidefolie 8 sowie der Flüssigkeitsauffangbeutel 9.

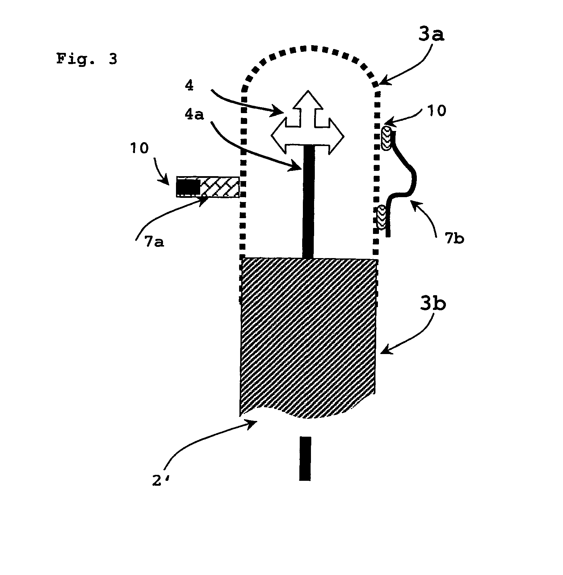

**Fig. 3** zeigt einen Schnitt durch eine weitere vorteilhafte Ausführungsform des Abdeckmittels 3, dargestellt mit Bezugsmittel 3 in Form eines dreiarmigen Zeigers, welcher auf einer Halterung 4a angebracht ist. An den oberen für die bekannten Vermessungsysteme transparenten Teil 3a schliesst sich ein nichttransparenter Teil 3b an. Dieser kann z.B. aus steiferem oder/und zäherem Material als der obere Teil gefertigt sein, so dass das gesamte Abdeckmittel 3 mehr oder weniger ohne eine Abstützung auf einer Halterung selbsttragend steht.

An der linken Seite ist ein Verjüngsmittel 7a dargestellt mit der der Umfang des Abdeckmittel senkrecht zur Längsachse verjüngt werden kann. Dazu ist in diesem Ausführungsbeispiel, d.h. nur beispielhaft ein Streifen mit einem Klebeelment 10 vorgesehen.

Zur Verkürzung des Abdeckmittels ist, auf der rechten Seite der Figur dargestellt, ein ebenfalls mit Klebelementen 10 ausgestattetes Verkürzungszmittel 7b vorgesehen. Somit kann das Abdeckmittel auf einfache Weise dem Bezugsmittel und dessen Lage (z.B. durch eine bewegliche Halterung variierbar) angepasst werden, ohne dass unnötige Falten die vom Vermessungssystem ausgehende Strahlung durch das Abdeckmittel oder die reflektierte Strahlung vom Bezugsmittel gestört wird (z.B. durch Streuung etc.).

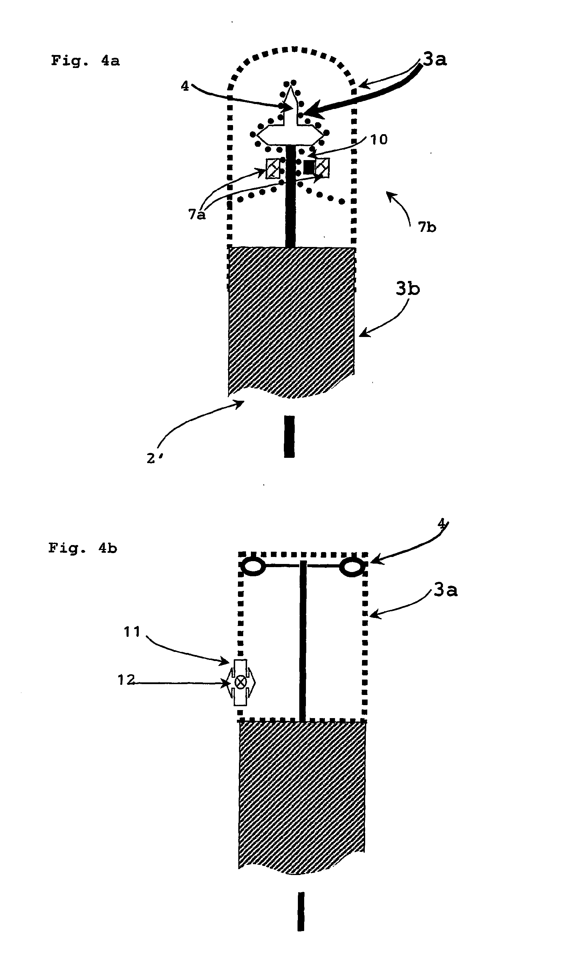

**Fig. 4a** zeigt das Abdeckmittel 3 in einer Form (runde Punkte) die durch die Verjüngungsmittel 7a an die Form des Bezugsmittels angepasst wurde.

Diese Anpassung kann natürlich auch durch eine Vor-Formung des Abdeckmittels geschehen, wozu das obere Ende des Abdeckmittels entsprechende Ausformungen aufweist.

**Fig. 4b** zeigt eine weiteres vorteilhaftes Ausführungsbeispiel des Abdeckmittels 3, wobei diesmal ein planares Ende für eine gute Passform an das in diesem Fall aus einem dreiarmigen Stern mit Kugeln als Bezugspunkten gebildete Bezugsmittel sorgt.

Eine ganz besonders bevorzugte Ausführungsform ist dadurch gegeben, dass das Abdeckmittel mit elastischen oder plastischen, transparenten Materialien ausgebildet ist, wobei das Abdeckmittel gleichzeitig auch aufblasbar ausgebildet ist. Damit wird jegliche Bildung von Falten etc. unterbunden. Dazu ist am Abdeckmittel das untere Ende geschlossen ausgeführt und weist an der Oberfläche ein Ventil und ggf. Pumpmittel auf.

Es ist unmittelbar ersichtlich, dass die Abdeckungsmittel nicht unbedingt gestreckt oder zylinderförmig mit einem oder zwei geschlossenen Enden ausgeführt sein muss. Jede andere Form kann ebenfalls zur gewünschten sterilen Abdeckung eines Bezugsmittels beitragen.

Unter Bezugsmittel wird hier jedes Mittel verstanden, welches durch die bekannten Vermessungs- oder Navigationssysteme im Krankenhausbetrieb dazu geeignet ist, einen Punkt im 2- oder 3-dimensionalen Raum zu markieren oder damit einen Koordinaten-Bezugspunkt zu verbinden.

### [Bezugszeichenliste]

- Abdecktuch: 1
- Fenestration: 2, 2', 2'', 2'''
- Abdeckmittel: 3
- Abdeckmittel, transparenter Bereich: 3a
- Abdeckmittel, nichttransparenter Bereich: 3b
- Bezugsmittel: 4
- Halterung für Bezugsmittel: 4b
- Chirurgischer Eingriffsbereich: 5
- Vermessungssystem, Navigationssystem: 6
- Verjüngungsmittel: 7a
- Verkürzungsmittel: 7b
- Einschneidefolie: 8
- Flüssigkeitsauffangbeutel: 9
- Klebeelement: 10
- Ventil: 11
- Pumpmittel: 12

## Patentansprüche

1. Chirurgisches Abdecktuch (1) mit einer ersten Fenestration (2) für eine Einschneidefolie (8) mit oder ohne einem dieser Folie zugeordneten Auffangbeutel (9) für Operationsrückstände, wobei das Abdecktuch (1) mindestens eine weitere Fenestration (2', 2", 2''') aufweist, **dadurch gekennzeichnet, dass** in die weitere Fenestration (2', 2", 2''') ein Abdeckmittel (3, 3a, 3b) für ein aus der Ebene der Oberfläche des Abdecktuches (1) herausragendes Bezugsmittel (4) aufgenommen ist, welches für ein 2 oder 3-dimensionales Vermessungssystem (6) identifizierbar ist.

2. Abdecktuch nach Anspruch 1, **dadurch gekennzeichnet, dass** das Abdeckmittel (3, 3a, 3b) mindestens im Bereich der Abdeckung des Bezugsmittels transparent für die von einem Vermessungssystem (6), insbesondere einem 2-oder 3-dimensionalen Infrarot-Vermessungssystem ausgesendete und vom Bezugsmittel (4) reflektierte Strahlung ist.

3. Abdecktuch nach Anspruch 1 bis 2, **dadurch gekennzeichnet, dass** das Abdeckmittel (3, 3a, 3b) mit dem Abdecktuch (1) fest verbunden oder verbindbar, z.B. durch Klebestreifen, entlang des gesamten Umfangs der zweiten Fenestration (2', 2", 2''') ausgeführt ist.

4. Abdecktuch nach Anspruch 1 bis 3, **dadurch gekennzeichnet, dass** das Abdeckmittel (3, 3a, 3b) aus einem flexiblen Material, z.B. aus einem Polymer ausgeführt ist.

5. Abdecktuch nach Anspruch 1 bis 4, **dadurch gekennzeichnet, dass** das Abdeckmittel (3, 3a, 3b) eine gestreckte oder streckbare Form mit einem verschlossenen Ende an der vom Abdecktuch (1) weggerichteten Seite, z.B. eine zylindrische Form aufweist.

6. Abdecktuch nach Anspruch 1 bis 5, **dadurch gekennzeichnet, dass** das Abdeckmittel (3, 3a, 3b) entlang der Oberfläche zwischen Abdecktuch (1) und oberem Ende mindestens ein Verjüngungsmittel (7a), zur Verjüngung des Umfangs des Abdeckmittels (1) ungefähr senkrecht zur Längsachse vom unteren, mit dem Abdecktuch (1) verbundenen zum oberen Ende aufweist.

7. Abdecktuch nach Anspruch 1 bis 6, **dadurch gekennzeichnet, dass** das Abdeckmittel (3, 3a, 3b) entlang der Oberfläche zwischen Abdecktuch (1) und oberem Ende mindestens ein Verjüngungsmittel (7b), zur Verkürzung der Länge des Abdeckmittels (3, 3a, 3b) vom unteren, mit dem Abdecktuch (1) verbundenen zum oberen Ende aufweist, so dass auch das obere Ende festanliegend und glatt über die Bezugsmittel (4) spannbar ist, wodurch Falten oder andere Störungen der auf die Bezugsmittel (4) fallenden oder davon reflektieren Strahlung des Vermessungssystems (6) vermieden werden.

8. Abdecktuch nach Anspruch 6 bis 7, **dadurch gekennzeichnet, dass** die Verjüngungsmittel (7a, 7b) in Form von abnehmbaren Klebestreifen oder einfachen Schnüren ausgeführt sind.

9. Abdecktuch nach Anspruch 1 bis 8, **dadurch gekennzeichnet, dass** das Abdeckmittel (3, 3a, 3b) an seinem oberen, dem Abdecktuch (1) gegenüberliegenden Ende vorgeformte Ausnehmungen zur Aufnahme von Ausformungen des Bezugsmittels (4), z.B. in Form von Kugeln aufweist.

10. Abdecktuch nach Anspruch 1 bis 9, **dadurch gekennzeichnet, dass** das Abdeckmittel (3, 3a, 3b), z.B. durch Gamma-Strahlung, Heissdampf oder andere im Stand der Technik bekannte Verfahren, sterilisierbar ausgeführt ist.

11. Abdecktuch nach Anspruch 1 bis 10, **dadurch gekennzeichnet, dass** das Abdecktuch (1) entlang der Fenestration (2', 2", 2''') durch die Technik des Ultraschallschweissens, Klebens oder Wärmeschweissens fest mit dem Abdeckmittel (3, 3a, 3b) verbunden ausgeführt ist, wobei der Saum des Abdeckmittels (3, 3a, 3b) - zur Vermeidung der Einleitung von unsterilen Materialien - vorzugsweise auf der oberen, d.h. der dem Bezugsmittel (4) zugewandten Oberfläche des Abdecktuches (1) angebracht ausgeführt ist.

12. Abdecktuch nach Anspruch 1 bis 11, **dadurch gekennzeichnet, dass** das Abdeckmittel (3, 3a, 3b) aus einem plastischem oder elastischem Material und insbesondere im Bereich der Abdeckung des Bezugsmittels (4), aufblasbar ausgeführt ist, so dass insbesondere im Bereich der Abdeckung des Bezugsmittels (4) eine Faltenbildung und damit eine Störung der auf das Bezugsmittel (4) fallenden oder von diesem reflektierten Strahlung vermieden wird.

13. Abdecktuch nach Anspruch 1 bis 12, **dadurch gekennzeichnet, dass** mindestens zwei Abdeckmittel (3, 3a, 3b) - in jeweils einer Fenestration (2', 2", 2''') des Abdecktuches (1) angebracht - vorgesehen sind, wobei die Abdeckmittel (3, 3a, 3b) einen minimalen Aussendurchmesser von 10 bis 50 cm, vorzugsweise 25 cm aufweisen.

14. Abdecktuch nach Anspruch 1 bis 13, **dadurch gekennzeichnet, dass** mindestens zwei Abdeckmittel (3, 3a, 3b) - jeweils in einer zweiten, dritten und weiteren Fenestration (2', 2", 2''') des Abdecktuches (1) angebracht - vorgesehen sind, wobei die Abdeckmittel (3, 3a, 3b), gemessen vom Flächenmittelpunkt der Fenestration (2', 2", 2''') am Fusse der Abdeckmittel (3, 3a, 3b), einen Abstand von 10 bis 100 cm, bevorzugt jedoch 50 cm vom Zentrum der Einschneidefolie (8) aufweisen.

15. Abdecktuch nach Anspruch 14, **dadurch gekennzeichnet, dass** jeweils eines der mindestens zwei Abdeckmittel (3, 3a, 3b) links und rechts ca. 40 cm im lotrechten Abstand vom Zentrum der Einschhneidefolie (8) beabstandet angeordnet sind, so dass auch während einer Operation leicht ein Umschwenken der Bezugsmittel (4) von einer zur anderen Seite, was meist in Verbindung mit einer Umlagerung des Patienten notwendig ist, vorgenommen werden kann.

## Claims

1. Surgical drape (1) with a first fenestration (2) for an incise film (8) with or without a collection pouch (9) for surgical residues related to said film, said drape (1) comprising at least one further fenestration (2', 2", 2''') wherein, in the further fenestration (2', 2", 2'''), a cover means (3, 3a, 3b) for a reference means (4) protruding from the level of the drape's surface is integrated, said reference means (4) being identifiable for a 2- or 3-dimensional measurement system (6).

2. Drape according to claim 1, wherein the cover means (3, 3a, 3b), at least in the area of coverage of the reference means (4), is transparent for radiation emitted by a measurement system (6), in particular from a 2- or 3-dimensional infrared measurement system and reflected from the reference means (4).

3. Drape according to claims 1 to 2, wherein the cover means (3, 3a, 3b) is realized to be firmly attached or able to be attached to the drape (1), e.g. by adhesive strips, along the entire perimeter of the second fenestration (2', 2", 2''').

4. Drape according to claims 1 to 3, wherein the cover means (3, 3a, 3b) is made of a flexible material, e.g. a polymer.

5. Drape according to claims 1 to 4, wherein the cover means (3, 3a, 3b) comprises a form which is stretched or able to be stretched having a closed end on the side facing away from the drape (1), e.g. a cylindrical form.

6. Drape according to claims 1 to 5, wherein the cover means (3, 3a, 3b) comprises along the surface between the drape (1) and the upper end at least one reduction means (7a), suitable for reducing the perimeter of the cover means (3) approximately vertically to the longitudinal axis from the bottom end, attached to the drape, to the upper end.

7. Drape according to claims 1 to 6, wherein the cover means (3, 3a, 3b) comprises at least one reduction means (7b) along the surface between the drape (1) and the upper end for reducing the length of the cover means (3, 3a, 3b) from the bottom end, attached to the drape (1), to the upper end, so that the upper end is also apt to be stretched firmly and smoothly over the reference means (4), avoiding creases or other distortions of the radiation of the measurement system (6) falling on the reference means (4) or reflected therefrom.

8. Drape according to claims 6 to 7, wherein the reduction means (7a, 7b) is realized in the form of removable adhesive strips or simple cords.

9. Drape according to claims 1 to 8, wherein the cover means (3, 3a, 3b) comprises pre-shaped recesses on its upper, drape(1)-opposing end for receiving shapes of the reference means (4), e.g. in the form of balls.

10. Drape according to claims 1 to 9, wherein the cover means (3, 3a, 3b) is suited to be sterilized, e.g. by gamma radiation, hot steam or other methods known in the state of the art.

11. Drape according to claims 1 to 10, wherein the drape (1) is firmly attached to the cover means (3, 3a, 3b) along the fenestration (2', 2", 2''') by means of ultrasonic welding, adhesion or heat welding, the border of the cover means (3, 3a, 3b) - in order to avoid penetration of non-sterile materials - being preferably attached to the upper surface, i.e. the surface of the drape (1) facing the reference means (4).

12. Drape according to claims 1 to 11, wherein the cover means (3, 3a, 3b) is realized with an elastic or plastic material and, is capable to be inflated in particular, in the area of the coverage of the reference means (4) thus avoiding, particularly in the area of the coverage of the reference means (4), the formation of creases and therefore the distortion of radiation falling on the reference means (4) or reflected therefrom.

13. Drape according to claims 1 to 12, wherein at least two cover means (3, 3a, 3b) - attached to one fenestration (2', 2", 2''') of the drape (1) respectively - are provided, the cover means (3, 3a, 3b) comprising a minimum outside diameter of 10 to 50 cm, preferably of 25 cm.

14. Drape according to claims 1 to 13, wherein at least two cover means (3, 3a, 3b) - attached to a second, third, and further fenestration (2', 2", 2''') of the drape (1), respectively - are provided, the cover means (3, 3a, 3b), measured from the middle point of the fenestration (2', 2", 2''') surface at the foot of the cover means (3, 3a, 3b), comprise a distance of 10 to 100 cm, preferably, however, 50 cm from the center of the incise film (8).

15. Drape according to claim 14, wherein one of the at least two cover means (3, 3a, 3b), respectively, is arranged at a distance of approx. 40 cm left and right in a perpendicular distance from the center of the incise film (8), so that during an operation swinging the reference means (4) from one side to the other can be easily realized as well, which is usually necessary in connection with the relocation of the patient.

## Revendications

1. Tissu opératoire chirurgical (1) avec une première fenestration (2) pour un film transparent à incision (8) avec ou sans poche de réception pour des résidus opératoires (9) accompagnant ce film transparent, le tissu opératoire (1) présentant au moins une fenestration supplémentaire (2', 2", 2'''), **caractérisé en ce qu'**un dispositif de recouvrement (3, 3a, 3b) est placé au niveau de la fenestration supplémentaire (2', 2", 2"') pour recevoir un dispositif de repérage (4) émergeant au niveau de la face extérieure du tissu opératoire (1), le dispositif de repérage étant identifiable pour un système de mesure en 2 ou 3 dimensions (6).

2. Tissu opératoire selon la revendication 1, **caractérisé en ce que** le dispositif de recouvrement (3, 3a, 3b) est transparent au moins dans le secteur de recouvrement du dispositif de repérage (4) pour le rayonnement émis d'un système de mesure (6), en particulier d'un système de mesure infrarouge en 2 ou 3 dimensions, et réfléchi par le dispositif de repérage (4).

3. Tissu opératoire selon les revendications 1 à 2, **caractérisé en ce que** le dispositif de recouvrement (3, 3a, 3b) est lié ou peut être lié d'une manière fixe au tissu opératoire (1), par exemple au moyen de bandes adhésives, le long de l'ensemble du périmètre de la deuxième fenestration (2',2", 2''').

4. Tissu opératoire selon les revendications 1 à 3, **caractérisé en ce que** le dispositif de recouvrement (3, 3a, 3b) est fabriqué d'un matériau flexible, par exemple d'un polymère.

5. Tissu opératoire selon les revendications 1 à 4, **caractérisé en ce que** le dispositif de recouvrement (3, 3a, 3b) présente une forme étirée ou étirable avec une extrémité close du côté opposé au tissu opératoire (1), par exemple une forme cylindrique.

6. Tissu opératoire selon les revendications 1 à 5, **caractérisé en ce que** le dispositif de recouvrement (3, 3a, 3b) présente le long de la surface entre le tissu opératoire (1) et l'extrémité supérieure au moins un dispositif de rétrécissement (7a), prévu pour le rétrécissement du périmètre du dispositif de recouvrement (3) quasiment perpendiculaire à l'axe de la longueur, de l'extrémité inférieure liée au tissu opératoire (1) à l'extrémité supérieure.

7. Tissu opératoire selon les revendications 1 à 6, **caractérisé en ce que** le dispositif de recouvrement (3, 3a, 3b) présente le long de la surface entre le tissu opératoire (1) et l'extrémité supérieure au moins un dispositif de rétrécissement (7b), prévu pour le raccourcissement de la longueur du dispositif de recouvrement (3, 3a, 3b), de l'extrémité inférieure liée au tissu opératoire (1) à l'extrémité supérieure, de manière à pouvoir tendre également l'extrémité supérieure sur les dispositifs de repérage (4) en la serrant de près et la lissant, ce qui empêche la formation de plis ou d'autres perturbations du rayonnement du système de mesure (6) tombant sur les dispositifs de repérage (4) ou réfléchi par celui-ci.

8. Tissu opératoire selon les revendications 6 à 7, **caractérisé en ce que** les dispositifs de rétrécissement (7a, 7b) prennent la forme de bandes adhésives retirables ou de simples cordons.

9. Tissu opératoire selon les revendications 1 à 8, **caractérisé en ce que** le dispositif de recouvrement (3, 3a, 3b) présente au niveau de son extrémité supérieure faisant face au tissu opératoire (1) des creux préformées pour l'accueil de formes du dispositif de repérage (4), par exemple de forme sphérique.

10. Tissu opératoire selon les revendications 1 à 9, **caractérisé en ce que** le dispositif de recouvrement (3, 3a, 3b) est stérilisable, par exemple au moyen de rayons gamma, de vapeur bouillante ou d'autres procédés connus au niveau de l'état actuel de la technique.

11. Tissu opératoire selon les revendications 1 à 10, **caractérisé en ce que** le tissu opératoire (1) est lié fixement au dispositif de recouvrement (3, 3a, 3b) au moyen de la technique de soudage par ultrason, du collage ou du soudage par chaleur, le long de la fenestration (2', 2", 2'''), le bord du dispositif de recouvrement (3, 3a, 3b) étant placé - afin d'éviter l'introduction de matières non stériles - de préférence sur la surface supérieure du tissu opératoire (1) c'est à dire la surface tournée vers le dispositif de repérage (4).

12. Tissu opératoire selon les revendications 1 à 11, **caractérisé en ce que** le dispositif de recouvrement (3, 3a, 3b) est fabriqué d'un matériau plastique ou élastique et notamment dans le secteur du recouvrement du dispositif de repérage (4) de manière à pouvoir être gonflé, pour empêcher en particulier dans le secteur du recouvrement du dispositif de repérage (4) la formation de plis et par conséquent d'une perturbation du rayonnement tombant sur le dispositif de repérage (4) ou réfléchi par celui-ci.

13. Tissu opératoire selon les revendications 1 à 12, **caractérisé en ce qu'**au moins deux dispositifs de recouvrement (3, 3a, 3b) - respectivement placés dans une fenestration (2', 2", 2"') du tissu opératoire (1) - sont prévus, les dispositifs de recouvrement (3, 3a, 3b) présentant un diamètre extérieur minimum de 10 à 50 cm, et de préférence de 25 cm.

14. Tissu opératoire selon les revendications 1 à 13, **caractérisé en ce qu'**au moins deux dispositifs de recouvrement (3, 3a, 3b) - respectivement placés dans une deuxième, troisième etc. fenestration (2', 2", 2''') du tissu opératoire (1) - sont prévus, les dispositifs de recouvrement (3, 3a, 3b), mesurés du centre de surface de la fenestration (2', 2", 2"') au pied des moyens de recouvrement (3, 3a, 3b), présentent une distance vers le centre du film transparent à incision (8) de 10 à 100 cm, et de préférence de 50 cm.

15. Tissu opératoire selon la revendication 14, **caractérisé en ce que** respectivement au moins un des deux dispositifs de recouvrement (3, 3a, 3b) est disposé de façon à respecter à gauche et à droite une distance perpendiculaire au centre du film transparent à incision (8) d'environ 40 cm, de manière à pouvoir procéder facilement même pendant une opération à un déplacement d'un côté à l'autre des moyens de repérage (4), ce qui est dans la plupart du temps nécessaire lorsqu'il faut bouger le patient.
